# EUROPEAN PATENT APPLICATION

(11) **EP 4 623 833 A1**
(43) Date of publication of application: **01.10.2025**
(21) Application number: 24305483.0
(22) Date of filing: 28.03.2024
(51) Int. Cl.: A61B 6/12, A61B 6/46, A61B 6/50, A61B 6/00, A61B 6/02, A61B 6/03

(54) **PROVIDING FEEDBACK DURING BALLOON ANGIOPLASTY**

(71) Applicant: Koninklijke Philips N.V., 5656 AG Eindhoven (NL)
(72) Inventor: HERMANS, Luc, 5656 Eindhoven (NL); GROEN, Joanneke, 5656 Eindhoven (NL); HENDRIKS, Bernardus, 5656 Eindhoven (NL); FLORENT, Raoul, 5656 Eindhoven (NL); OLIVAN BESCOS, Javier, 5656 Eindhoven (NL); AUVRAY, Vincent, 5656 Eindhoven (NL); LUCASSEN, Gerald, 5656 Eindhoven (NL); LARUE, Ruben, 5656 Eindhoven (NL); LAI, Marco, 5656 Eindhoven (NL)
(74) Representative: Philips Intellectual Property & Standards

(57) **Abstract**

A system for providing feedback during balloon angioplasty, is provided. The system comprises one or more processors configured to: receive projection image data representing a balloon in a vessel; and output, based on a shape (140) of the balloon in a simulated expanded state in the vessel, and based on a shape (150) of the balloon represented in the projection image data, information (160, 170) for comparing a state of expansion of the balloon represented in the projection image data with the simulated expanded state.

## Description

### TECHNICAL FIELD

The present disclosure relates to providing feedback during balloon angioplasty. A system, a computer-implemented method, and a computer program product, are disclosed.

### BACKGROUND

Balloon angioplasty, also known as percutaneous transluminal angioplasty "PTA", is a medical procedure in which a balloon is expanded within a blood vessel in order to dilate the vessel lumen and thereby increase blood flow through the vessel. Balloon angioplasty is performed on blood vessels that have become narrowed as a result of conditions such as atherosclerosis, i.e. the buildup of plaque in a blood vessel. Balloon angioplasty is often performed in combination with a stent. Stenting refers to a balloon angioplasty procedure wherein the balloon is expanded within a stent, which in turn dilates the vessel lumen. The stent is left in the vessel in order to support the vessel lumen in the dilated state and thereby prevent its subsequent collapse. Balloon angioplasty is performed on arteries as well as veins. Balloon angioplasty is performed in various parts of the anatomy, including the heart, the brain, and also in peripheral regions such as the leg, the kidney, and so forth.

However, during balloon angioplasty, physicians face a challenge in determining whether a vessel lumen has been sufficiently dilated in order for the procedure to be successful. For instance, when treating a calcified lesion with balloon angioplasty, calcium deposits within the lesion affect the balloon's expansion. As the balloon expands, it ruptures the calcium deposits, pushing them to one side. However, calcium deposits are often distributed asymmetrically around the vessel lumen. The calcium deposits lead to an asymmetrical expansion of the balloon. In such situations it is difficult for the physician to assess whether the vessel lumen has been sufficiently dilated.
Consequently, there is a need to provide improved feedback during balloon angioplasty.

### SUMMARY

According to one aspect of the present disclosure, a system for providing feedback during balloon angioplasty, is provided. The system includes one or more processors configured to:
receive projection image data representing a balloon in a vessel;
output, based on a shape of the balloon in a simulated expanded state in the vessel, and based on a shape of the balloon represented in the projection image data, information for comparing a state of expansion of the balloon represented in the projection image data with the simulated expanded state.

Since the information for comparing a state of expansion of the balloon represented in the projection image data with the simulated expanded state is determined based on a shape of the balloon in a simulated expanded state in the vessel, the system provides a reliable assessment of the state of expansion of the balloon represented in the projection image data. Consequently, the system provides reliable feedback on the progress of the balloon angioplasty.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for providing feedback during balloon angioplasty, in accordance with some aspects of the present disclosure.
Fig. 2 is a flowchart illustrating an example of a computer-implemented method of providing feedback during balloon angioplasty, in accordance with some aspects of the present disclosure.
Fig. 3 is an example of projection image data 120 representing a balloon 130 in a vessel, in accordance with some aspects of the present disclosure.
Fig. 4 illustrates a first example of outputted information 160, 170 for comparing a state of expansion of a balloon represented in projection image data with a simulated expanded state, in accordance with some aspects of the present disclosure.
Fig. 5 illustrates a second example of outputted information 160, 170 for comparing a state of expansion of a balloon represented in projection image data with a simulated expanded state, in accordance with some aspects of the present disclosure.
Fig. 6 is a schematic diagram illustrating an example of the generation of a first graphical representation 140 comprising a virtual projection of a balloon 130^{VP}, in accordance with some aspects of the present disclosure.
Fig. 7 illustrates an example of a recommended viewing angle, α₁, for visualizing a calcium deposit in a vessel, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in one example, and that all features are not necessarily duplicated in each example for the sake of brevity. For instance, features described in relation to a computer-implemented method, may be implemented in a computer program product, and in a system, in a corresponding manner.

In the following description, reference is made to examples of a system for providing feedback during balloon angioplasty. In some examples, reference is made to a balloon angioplasty procedure that is performed on an artery of the heart. It is, however, to be appreciated that the artery, and the heart, serve only as examples. In general, the system may be used to provide feedback during balloon angioplasty procedures that are performed on any type of blood vessel, and the blood vessel may be in any anatomical region. For instance, the system may be used to provide feedback during balloon angioplasty procedures that are performed on artery, or a vein, and the blood vessel may be in the heart, the brain, or in a peripheral region such as the leg, the kidney, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is a need to provide improved feedback during balloon angioplasty.

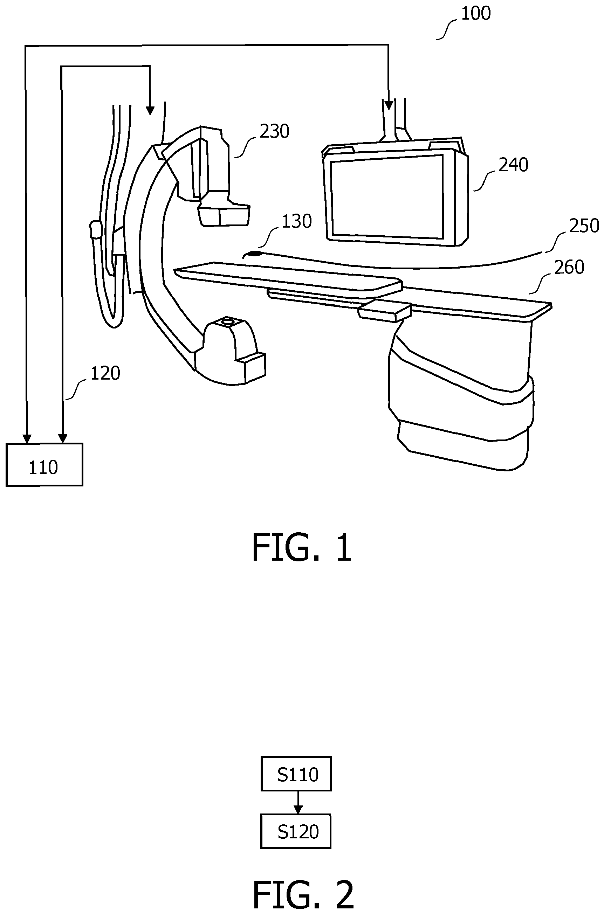

Fig. 1 is a schematic diagram illustrating an example of a system 100 for providing feedback during balloon angioplasty, in accordance with some aspects of the present disclosure. Fig. 2 is a flowchart illustrating an example of a computer-implemented method of providing feedback during balloon angioplasty, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for providing feedback during balloon angioplasty comprises one or more processors 110 configured to:
receive S110 projection image data 120 representing a balloon 130 in a vessel;
output S120, based on a shape 140 of the balloon in a simulated expanded state in the vessel, and based on a shape 150 of the balloon 130 represented in the projection image data 120, information 160, 170 for comparing a state of expansion of the balloon 130 represented in the projection image data 120 with the simulated expanded state.

Since the information 160, 170 for comparing a state of expansion of the balloon 130 represented in the projection image data 120 with the simulated expanded state is determined based on a shape 150 of the balloon 130 in a simulated expanded state in the vessel, the system 100 provides an reliable assessment of the state of expansion of the balloon represented in the projection image data. Consequently, the system 100 provides reliable feedback on the progress of the balloon angioplasty.

The operations that are performed by the one or more processors 110 of the system 100 are described in more detail below.

In the operation S110, projection image data 120 is received.

The projection image data 120 that is received in the operation S110 may be generated by a projection X-ray imaging system . The projection X-ray imaging system may be a spectral projection X-ray imaging system. Spectral projection X-ray imaging systems generate X-ray attenuation data representing X-ray attenuation within multiple different energy intervals. The X-ray attenuation data generated by a spectral projection X-ray imaging system may be processed, e.g. using various material decomposition algorithms, in order to distinguish between media that have similar X-ray attenuation values when measured within a single energy interval, and which would be indistinguishable in X-ray attenuation data obtained using a conventional projection X-ray imaging system. Thus, X-ray attenuation data generated by a spectral projection X-ray imaging system, may be processed to provide projection images with improved specificity to materials such as a vessel wall, plaque components, contrast agent, tissue, bone, and so forth.

The projection image data 120 that is received in the operation S110 represents a balloon 130 in a vessel.

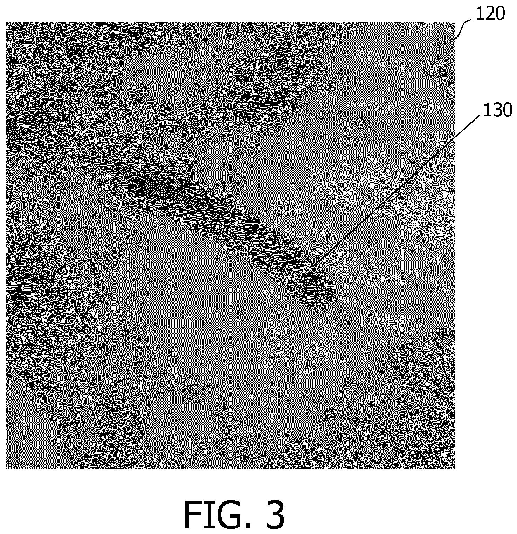

The projection image data 120 may represent various type of balloons. By way of some examples, the balloon may be a compliant balloon, a non-compliant balloon, an "over-the wire" balloon, a rapid exchange balloon, a balloon comprising scoring wires or blades, and so forth. In general, the balloon may be expandable via the injection of a gas, such as air, or a liquid, into the balloon. Fig. 3 is an example of projection image data 120 representing a balloon 130 in a vessel, in accordance with some aspects of the present disclosure. In the example illustrated in Fig. 3, the projection image data 120 is illustrated in the form of an X-ray projection image. The balloon 130 is filled with a liquid that includes a contrast agent. The contrast agent enhances the balloon's visibility in the X-ray projection image. Some balloons also include one or more radiopaque markers that serve to further enhance their visibility in X-ray projection images. The radiopaque markers may be disposed at the axial ends of the balloon, for example.

In the example illustrated in Fig. 3, the balloon 130 is in a partially-expanded state and the projected shape of the balloon is visible in the X-ray projection image. During angioplasty, a pressure in the balloon 130 is increased. The pressure causes the balloon to expand, thereby exerting a dilating force on media surrounding the balloon, such as the vessel wall, and also any plaque that may be within the vessel. When the vessel lumen has been sufficiently dilated, the pressure in the balloon is reduced and the balloon contracts. In some situations, a balloon is expanded and contracted only once in order to achieve the desired amount of vessel lumen dilation. In other situations, a balloon is expanded and contracted multiple times. After the vessel lumen has been dilated, a check-up may be performed wherein a contrast agent is injected into the vasculature and an angiogram acquired in order to verify the amount of vessel lumen dilation that has been achieved.

With continued reference to the operation S110, the projection image data 120 that is received in the operation S120 may be received from various sources. For instance, it may be received from a (spectral) projection X-ray imaging system, described above. In the example illustrated in Fig. 1, the projection image data 120 is received from the projection X-ray imaging system 230.

In general, the projection image data 120 that is received in the operation S110 may be received by the one or more processors 110 via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 illustrated in Fig. 1; in this operation, information 160, 170 is outputted for comparing a state of expansion of the balloon 130 represented in the projection image data 120 with a simulated expanded state. The outputted information is based on a shape 140 of the balloon in the simulated expanded state in the vessel, and based on a shape 150 of the balloon 130 represented in the projection image data 120.

The simulated expanded state may refer to any state of expansion of the balloon. The simulated expanded state may be a partially-expanded state, or a fully expanded state, for example. The shape 140 of the balloon in the simulated expanded state may be determined using various techniques in the operation S120. For instance, a model, such as a finite element model, or a machine learning technique, may be used to simulate the shape of the balloon in the expanded state. The interventionalist might also directly input some desired shapes or characteristics that can be taken into account to produce the simulated expanded state.

In the former case, the shape of the balloon in the simulated expanded state may be determined based on image data representing the vessel. For example, volumetric image data representing the vessel may be acquired for the vessel, and the volumetric image data used to construct a geometrical model of the vessel. The volumetric image data may be (spectral) CT image data, or coronary computed tomography angiography "CCTA" data, or magnetic resonance imaging "MRI" data, 3D ultrasound image data, intravascular ultrasound "IVUS" image data, or optical coherence tomography "OCT" image data, for example. The volumetric image data may be segmented in order to provide a distinction between media such as the vessel, the vessel wall, and any plaque components that may be within the vessel. For example, CT image data may be segmented based on (Hounsfield Units) attenuation values in order to distinguish between such media. MRI image data may similarly be used to distinguish between such media. Material properties (e.g. stiffness, elasticity) for the vessel wall, and any plaque components within the vessel (e.g. plaque, calcified plaque, cholesterol, fibrin, and so forth) may then be assigned to the relevant materials. Alternatively, the motion of structures such as the vessel wall may be analyzed in temporal CT image data, or temporal MRI data, in order to extract their corresponding material properties. A geometrical model representing the balloon may similarly be constructed. The balloon may be modelled using known dimensions for the balloon, and known material properties, e.g. for its elasticity. A finite element simulation may then be performed using the geometrical model of the balloon within the geometrical model of the vessel in order to determine shape of the balloon in a simulated expanded state in the vessel.

In the latter case, a machine learning technique, such as a neural network, may be used to determine the shape of the balloon in the simulated expanded state. In this case, image data representing the vessel may be inputted into the machine learning model, and in response, the machine learning model predicts the shape of the balloon for a specified (e.g. partially, of fully) expanded state. The image data may include the projection image data 120 and/or the volumetric image data described above. The training data for training the machine learning model may include image data representing balloons in vessels during angioplasty procedures wherein the balloons have been expanded to different states of expansion.

In general, the simulations described above may be performed prior to the angioplasty procedure, for example during a planning phase, or alternatively they may be performed during the angioplasty procedure itself.

In one example, the shape 140 of the balloon in the simulated expanded state is determined using the one or more processors 110. In this example, the one or more processors 110 are configured to:
receive volumetric image data 180 representing the vessel; and
simulate, using the volumetric image data 180, a shape of an expanded virtual balloon 130^{V} in the vessel to provide the shape 140 of the balloon in the simulated expanded state.

In this example, the shape 140 of the balloon in the simulated expanded state may be determined using the model, or the machine learning technique described above. The volumetric image data may be generated by various types of volumetric imaging systems, such as a (spectral) CT imaging system, or an MRI system, or a 3D ultrasound imaging system, for example. The shape 140 of the balloon in the simulated expanded state may be determined based on various balloon parameters. For instance, the shape 140 of the balloon in the simulated expanded state may be determined based on balloon parameters such as balloon dimensions, balloon elasticity, balloon pressure, and so forth. The balloon parameters may be determined from a database that stores the balloon parameters for multiple balloon types. Alternatively the balloon parameters may be provided by a user. In the latter case, the one or more processors 110 may receive user input representing one or more balloon parameters for the virtual balloon; and the operation of simulating a shape of an expanded virtual balloon 130^{V} in the vessel may be performed using the balloon parameters.

In one example, the shape 140 of the balloon in the simulated expanded state in the vessel represents the shape of the balloon in the simulated expanded state within a virtual stent in the vessel.

In this example, the shape of the balloon is determined subject to the constraints of the stent. Thus, it provides the balloon shape during angioplasty procedures that are performed in combination with a stent. The shape of the balloon in the simulated expanded state within a virtual stent in the vessel may be determined by including in the simulation described above, a geometrical model of a stent, or by training the machine learning model using training data that includes image data representing balloons within stents in vessels during angioplasty procedures wherein the balloons have been expanded to different states of expansion.

In one example, the operation of simulating a shape of an expanded virtual balloon 130^{V} in the vessel to provide the shape 140 of the balloon in the simulated expanded state, comprises:
simulating, using the volumetric image data 180, a rupture of a calcium deposit 190 within the vessel; and
determining the shape 140 of the balloon in the simulated expanded state based on the simulated rupture of the calcium deposit 190.

The rupture of a calcium deposit may be simulated using the volumetric image data 180 described above. For instance, in one example, the operation of simulating a shape of an expanded virtual balloon 130^{V} in the vessel comprises:
analyzing the volumetric image data 180 to identify i) a wall 210 of the vessel, and ii) one or more plaque components 190, 220 within the vessel;
assigning mechanical parameters to i) the wall 210 of the vessel, and to ii) each of the one or more plaque components 190, 220 within the vessel; and
determining the shape of the expanded virtual balloon 130^{V} in the vessel based on i) the identified wall 210 of the vessel, and ii) the one or more plaque components 190, 220 within the vessel, using the assigned mechanical parameters.

As mentioned above, as the balloon expands during angioplasty, it ruptures calcium deposits, pushing them to one side. However, calcium deposits are often distributed asymmetrically around the vessel lumen. The calcium deposits lead to an asymmetrical expansion of the balloon. In such situations it is difficult for the physician to assess whether the vessel lumen has been sufficiently dilated. By determining the shape 140 of the balloon in the simulated expanded state based on the simulated rupture of the calcium deposit 190, this example provides an accurate simulated shape of the balloon following the rupture of the calcium deposit.

In this example, the operation of analyzing the volumetric image data may include assigning material types to image regions represented in the volumetric image data based on their (Hounsfield Units) X-ray attenuation values. The one or more plaque components may include plaque, calcified plaque, i.e. a calcium deposit, and so forth. For instance, calcium deposits may be distinguished from other plaque components and the vessel wall due to their relatively higher (Hounsfield Units) X-ray attenuation values. Similarly, the other plaque components may be distinguished from the vessel wall, based on their relatively higher (Hounsfield Units) X-ray attenuation values. The assigned mechanical parameters may include elasticity values, breaking stress values, and so forth. The assigned mechanical parameters may be used in the finite element simulation described above to determine the shape of the expanded virtual balloon 130^{V} in the vessel. The assigned mechanical parameters may also be used to evaluate the stress distribution in calcium deposits in relation the breaking stress. The stress distribution may be used to determine the state of expansion of the ballon at which the balloon causes a calcium deposit to rupture.

With continued reference to the operation S120 illustrated in Fig. 1, in this operation, information 160, 170, is outputted for comparing a state of expansion of the balloon 130 represented in the projection image data 120 with the simulated expanded state. The outputted information is based on a shape 140 of the balloon in a simulated expanded state in the vessel, and based on a shape 150 of the balloon 130 represented in the projection image data 120.

The information 160, 170 may be outputted in various forms in the operation S120. For instance, the information may be outputted in the form of a graphical representation, or as text, or an icon. The information may be outputted in various ways in the operation S120. For instance, the information may be outputted to a display, such as the display 240 illustrated in Fig. 1, or to a virtual/ augmented reality display device, or to printer, or to a computer-readable storage medium, or to the Internet, or to the Cloud, and so forth.

In one example, the outputting S120 information 160, 170 for comparing a state of expansion of the balloon 130 represented in the projection image data 120 with the simulated expanded state, comprises:
outputting a first graphical representation 140 of the balloon wherein the balloon is in the simulated expanded state; and
outputting a second graphical representation 150 of the balloon 130 wherein the balloon is in the state of expansion represented in the projection image data 120.

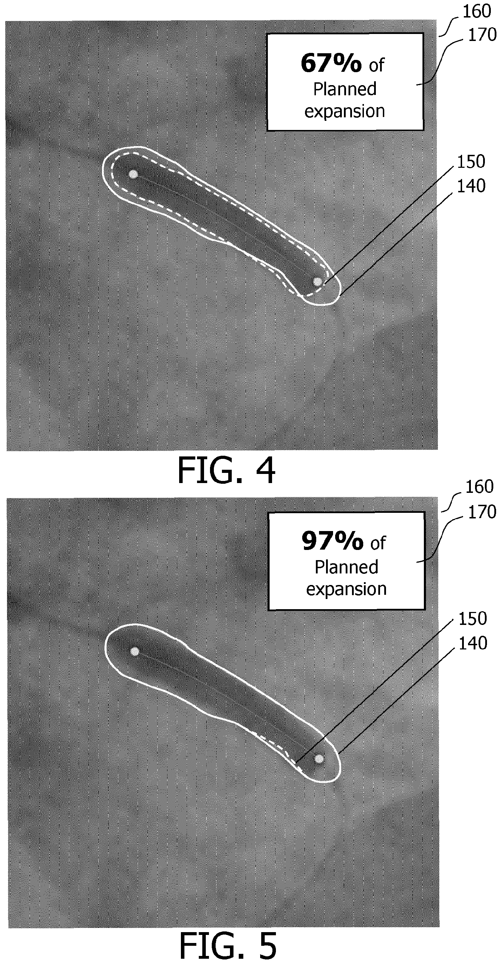

This example is described with reference to Fig. 4, and Fig. 5. Fig. 4 illustrates a first example of outputted information 160, 170 for comparing a state of expansion of a balloon represented in projection image data with a simulated expanded state, in accordance with some aspects of the present disclosure. Fig. 5 illustrates a second example of outputted information 160, 170 for comparing a state of expansion of a balloon represented in projection image data with a simulated expanded state, in accordance with some aspects of the present disclosure. The example illustrated in Fig. 5 represents a later stage in the expansion of the ballon than that illustrated in Fig. 4.

In the examples illustrated in Fig. 4 and Fig. 5, the first graphical representation 140 of the balloon includes an outline that corresponds to the projected shape 140 of the balloon in the simulated expanded state in the vessel. This outline may be determined using image processing techniques, as described in more detail below. In the examples illustrated in Fig. 4 and Fig. 5, the second graphical representation 150 of the balloon 130 includes an outline that corresponds to the shape 150 of the balloon in the projection image data 120. This outline may be determined by segmenting the projection image data. Various segmentation technique may be used for this purpose, such as model-based segmentation, watershed-based segmentation, region growing, level sets, graphcuts, and neural networks, for example.

In the examples illustrated in Fig. 4 and Fig. 5, the graphical representations of the balloons are displayed as an overlay on the projection image data 120. However, it is noted that the graphical representations may alternatively be displayed in a different manner. For instance, the outlines of the shapes of the balloons may be displayed without being overlaid onto the projection image data 120, or the outline of the shape 140 of the balloon in the simulated expanded state may simply be overlaid onto the projection image data 120. Instead of registering the balloons to one another as illustrated in Fig. 4 and Fig. 5, the first graphical representation 140 of the balloon, and the second graphical representation 150 of the balloon 130, may be displayed in a different manner. For instance, they may be displayed side-by-side.

In one example, the one or more processors 110 are configured to output one or more further graphical representations of the balloon wherein the balloon is in one or more further simulated expanded states. As mentioned above, the simulated expanded state may refer to any state of expansion of the balloon. The simulated expanded state may be a partially-expanded state, or a fully expanded state, for example. The graphical representations may represent the progressive expansion of the balloon throughout a series of simulated expanded states. Such graphical representations enable a physician to confirm that the shape of the balloon is expanding in accordance with their expectations at multiple points in time during the angioplasty procedure.

In one example, the first graphical representation 140 of the balloon represents the vessel and the calcium deposit 190, and the operation S120 of outputting information 160, 170 for comparing a state of expansion of the balloon 130 represented in the projection image data 120 with the simulated expanded state, includes outputting an indication of a location of the simulated rupture of the calcium deposit. This example may be used in combination with the example in which the rupture of a calcium deposit 190 within the vessel is simulated. In this example, the location of the simulated rupture of the calcium deposit may be indicated by highlighting the first or second graphical representation with a color, or a marker, for example.

In one example, the operation S120 of outputting information 160, 170 for comparing a state of expansion of the balloon 130 represented in the projection image data 120 with the simulated expanded state, includes outputting a value of a metric quantifying the simulated rupture of the calcium deposit 190. This example may be used in combination with the example in which the rupture of a calcium deposit 190 within the vessel is simulated. In this example, the metric may represent a change in calcium burden, or a total number of fragments resulting from the simulated rupture of the calcium deposit 190, or a maximum size of the fragments resulting from the simulated rupture of the calcium deposit 190, for example.

In one example, the first graphical representation 140 of the balloon represents the vessel, and the first graphical representation 140 comprises a distinction between the wall 210 of the vessel and at least one of one or more plaque components 190, 220.

In this example, a distinction between the wall 210 of the vessel and at least one of the one or more plaque components 190, 220 may be determined using the volumetric image data. For instance, if the volumetric image data represents (Hounsfield Units) X-ray attenuation values, then the (Hounsfield Units) X-ray attenuation values may be used to distinguish between these media. The distinction may be provided in the first graphical representation or the second graphical representation by rendering the wall 210 of the vessel and the at least one of the one or more plaque components 190, 220 with different colors, different shading, or different markers or icons, for example.

In one example, the balloon in the first graphical representation 140 is registered to the balloon in the second graphical representation 150. This arrangement is illustrated in Fig. 4 and Fig. 5. Registering the balloons to one another facilitates an accurate comparison of their shapes. The registration may be performed using various image processing techniques. For instance, if the viewing angle of the projection imaging system that was used to acquire the projection image data, is known, then the known viewing angle may be used to generate a projection of the volumetric image data 180 from the same viewing angle. The viewing angle may be determined by attaching an optical tracking system to the source-detector arrangement of the projection X-ray imaging system 230 illustrated in Fig. 1, and using the optical tracking system to track patient landmarks, or fiducial markers disposed on the patient. The patient landmarks, or fiducial markers, can be used to provide a 3D mesh defining a surface of the patient, and consequently they provide a correspondence between the coordinate system of the projection X-ray imaging system and the volumetric image data. Alternatively, an iterative technique that is based on finding an optimal fit between projections of the volumetric image data and the projection image data 120 may be used to determine a viewing angle with respect to the volumetric image data 180 from which the projection image data was obtained. This technique may include acquiring the projection image data from multiple angles with respect to a subject in order to constrain the fitting process.

In one example, the operation of outputting S120 information 160, 170 for comparing a state of expansion of the balloon 130 represented in the projection image data 120 with the simulated expanded state, comprises outputting a value of a metric 170 representing a difference between the shape 140 of the balloon in the simulated expanded state and the shape 150 of the balloon 130 represented in the projection image data 120.

The metric that is provided in accordance with this example provides feedback to a physician that enables the physician to determine when the simulated expanded state of the balloon has been reached. By way of some examples, the difference may be a difference in a volume of the balloons, or a difference in a surface area of the balloons, or a (maximum) difference in a cross sectional area of the balloons, or a (maximum) difference in a diameter of the balloons. In one example, the projection image data 120 is acquired from multiple viewing angles with respect to the balloon, and the difference is evaluated based on the projection image data that is acquired from the multiple viewing angles. For example, a surface of the balloon may be estimated from the projection image data by fitting a mesh to projection image data that is acquired from the multiple viewing angles. The surface of this mesh may then be fitted to the simulated shape of the balloon that is obtained from volumetric image data. This improves the accuracy of the difference that is evaluated in this example.

In this example, the shape 140 of the balloon in the simulated expanded state in the vessel may correspond to a planned state of expansion. In this case, the metric represents a fraction of the planned state of expansion that has been reached, or a remaining amount of expansion that is required until the planned state of expansion is reached. This example is illustrated in the upper right-hand portions of Fig. 4 and Fig. 5 via the label "67% of Planned expansion", and "97% of Planned expansion", respectively. This metric may be outputted in the operation S120 in combination with the first and second graphical representations of the balloon, i.e. in the manner illustrated in Fig. 4 and Fig. 5. Alternatively, this metric may be outputted instead of these graphical representations. In other words, text, or an icon, that represents this value may be outputted, e.g. to the display device 240 illustrated in Fig. 1.

In a related example, the operation of outputting S120 information 160, 170 for comparing a state of expansion of the balloon 130 represented in the projection image data 120 with the simulated expanded state comprises outputting an indication of a completion of a balloon angioplasty procedure based on the value of the metric 170 in relation to a threshold value. For example, the system 100 may output an indication that the balloon angioplasty procedure has been completed if the state of expansion of the balloon 130 represented in the projection image data 120 has exceeded a threshold value of 90%, 95%, 98%, and so forth.

In one example, the first graphical representation 140 comprises a virtual projection of the balloon 130^{VP}. The virtual projection of the balloon is generated by projecting the shape of the expanded virtual balloon 130^{V} simulated using the volumetric image data 180 such that the balloon 130^{VP} in the first graphical representation 140 is represented from a same viewing angle α₁ as the balloon 130 in the second graphical representation 150.

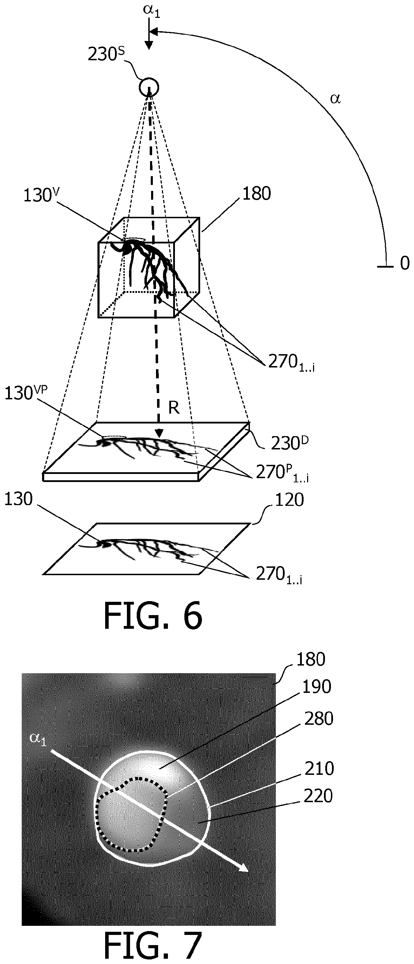

This example is illustrated in Fig. 6, which is a schematic diagram illustrating an example of the generation of a first graphical representation 140 comprising a virtual projection of a balloon 130^{VP}, in accordance with some aspects of the present disclosure. In the center of Fig. 6, the volumetric image data 180 represents the left branch of the coronary tree. The left branch of the coronary tree includes multiple vessels 270_{1..i}, including the left anterior descending artery "LAD". The volumetric image data is used to simulate the shape of an expanded virtual balloon 130^{V} in the LAD using the techniques described above. In the lower portion of Fig. 6, the projection image data 120 is illustrated. The projection image data includes the balloon 130, and also multiple vessels 270_{1..i}, including the LAD. The projection image data is generated by a projection X-ray imaging system that has a viewing angle with respect to the balloon. The shape of the expanded virtual balloon 130^{V} is projected, mathematically, onto the virtual X-ray detector 230^{D} using virtual X-rays that are emitted from a virtual X-ray source 230^{S}. This is illustrated in Fig. 6 for the virtual X-ray, R. Performing the ray tracing operation on the expanded virtual balloon 130^{V} results in the virtual projection of the balloon 130^{VP}. One or more of the vessel(s) 270_{1..i} represented in the volumetric image data 180 may also be projected in this operation to provide projected vessels 270^{P}_{1..i}, as illustrated in Fig. 6.

The shape of the expanded virtual balloon 130^{V} is projected such that the balloon 130^{VP} in the first graphical representation 140 is represented from a same viewing angle, α₁, as the balloon 130 in the second graphical representation 150. This operation may be performed based on a known correspondence between the viewing angle that is used to obtain the projection image data 120, and a reference orientation with respect to the volumetric image data. For example, the optical tracking system described above may be used to provide a correspondence between the coordinate system of the projection X-ray imaging system and the volumetric image data.

In one example, the volumetric image data 180 is used to recommend a viewing angle for acquiring the projection image data 120. In this example, the one or more processors 110 are configured to:
determine, based on the volumetric image data 180, a value of a recommended viewing angle α₁ for acquiring the projection image data 120; and
output the value of the recommended viewing angle α₁.

The recommended viewing angle may be determined based on various factors. For instance it may be based on one or more of: providing minimal foreshortening of the balloon 130^{VP} in the first graphical representation 140, providing optimal visualization of a calcium deposit in the vessel, maximizing a visualization of stenosis asymmetry, providing a minimum total number of vessels in the volumetric image data 180 overlapping with the stenosis. Such factors may be modelled using the volumetric image data 180 and weighted in order to determine the recommended viewing angle.

Fig. 7 illustrates an example of a recommended viewing angle, α₁, for visualizing a calcium deposit in a vessel, in accordance with some aspects of the present disclosure. Fig. 7 illustrates a view of the volumetric image data 180 along an axis of a vessel that includes a calcium deposit 190 and a non-calcium plaque component 220. The vessel wall 210 surrounds the calcium deposit 190 and the non-calcium plaque component 220. The calcium deposit 190 appears as a bright white region by virtue of its relatively higher X-ray attenuation as compared to non-calcium plaque. The vessel lumen 280 is also illustrated in Fig. 7. In this example, the recommended viewing angle, α₁, is optimized based on a distribution of the calcium deposit 190 with respect to a centerline of the vessel. The recommended viewing angle, α₁, may be chosen such that for a portion of the vessel corresponding to at least the axial extent of the balloon, the calcium deposit 190 is maximally asymmetrically distributed with respect to a centerline of the vessel, for example. Thus, at the recommended viewing angle, the calcium deposit 190 may lie to one side of the vessel centerline, as illustrated in Fig. 7.

The outputted value of the recommended viewing angle α₁ may be used to acquire the projection image data 120 that is received in the operation S110. For example, subsequent to the outputting of the value of the recommended viewing angle, α₁, a user may adjust the viewing angle α of a projection imaging system 230 to the recommended viewing angle α₁, or the one or more processors 110 of the system 100 may automatically adjust the viewing angle α to the recommended viewing angle α₁, prior to acquiring the projection image data 120.

In one example, the one or more processors 110 are configured to:
output an indication of a recommended type of a stent for use in stenting the vessel; and/or
output an indication of a recommended value of a dimension of a stent for use in stenting the vessel.

The type of the stent that is outputted in this example may refer to a manufacturer, or a model number of the stent. The dimension of the stent may refer to a length and/or a diameter and/or a mesh size of the stent. The recommended type and/or value of a dimension of the stent may be determined from the volumetric image data 180. For instance, a location of a stenosis may be identified in the vessel, automatically, or based on user input, and a diameter of the vessel proximal or distal to the stenosis may be used to determine a recommended value for the stent diameter. A length of the stenosis may be used to determine a recommended value for the stent length. A recommended mesh size of the stent may be determined based on a distribution of calcium deposit(s) along the stenosis.

In general, the projection image data 120 that is received in the operation S110 may include a single static image representing the balloon 130 in the vessel, or it may include a live sequence of images representing the balloon 130 in the vessel. In the former case, the outputting operation S120 is performed for the single static image. A subsequent static image may then be received, and the outputting again performed for the subsequent static image. In the latter case, the projection image data 120 comprises a live sequence of images representing the balloon 130 in the vessel, and the outputting S120 is performed for a current image in the live sequence and repeated for subsequent current images in the live sequence. The live sequence of images may be fluoroscopic X-ray images, for example.

As may be appreciated, in situations in which the projection image data 120 comprises a live sequence of images representing the balloon 130 in the vessel, the balloon can undergo significant motion. For instance, if the vessel is located in the heart, the balloon will move in accordance with the beating of the heart. This presents a significant challenge in tracking the progress of angioplasty procedures because the motion makes it difficult to visualize the current state of expansion of the ballon whilst the balloon is moving. Some examples of the system are described below that address the challenges presented by such cardiac motion.

In one example, the volumetric image data 180 represents the vessel at a cardiac phase. In this example, the operation of outputting S120 information 160, 170 for comparing a state of expansion of the balloon 130 represented in the projection image data 120 with the simulated expanded state, is performed selectively for current images from the live sequence representing the vessel at the cardiac phase represented in the volumetric image data 180.

Performing the outputting selectively for current images from the live sequence representing the vessel at the cardiac phase represented in the volumetric image data 180, restricts the outputting to images wherein the balloon is in a similar position. This reduces the impact of cardiac motion on the outputting, helping to provide a clearer comparison between the state of expansion of the balloon represented in the projection image data, and the simulated expanded state. In this example, the cardiac phase may be the end of the diastole phase, or the end of the systole phase, or an intermediate cardiac phase between systole and diastole, for example. The cardiac phase may be determined by analyzing the projection image data 120. For instance, the cardiac phase may be determined based on a movement of a portion of the heart in the projection image data. Alternatively, electrocardiogram "ECG" signals may be acquired during acquisition of the projection image data in order to determine the cardiac phase.

In another example, the operation of outputting S120 information 160, 170 for comparing a state of expansion of the balloon 130 represented in the projection image data 120 with the simulated expanded state, comprises:
registering the vessel at one or more different cardiac phases in the current images in the live sequence to the vessel at the cardiac phase represented in the volumetric image data 180; and
generating the outputted information 160, 170 based on the registered vessels.

In this example, the registering operation has the effect of spatially aligning the balloon in the current images to the balloon in the volumetric image data 180. As in the previous example, this also reduces the impact of cardiac motion on the outputting, helping to provide a clearer comparison between the state of expansion of the balloon represented in the projection image data, and the simulated expanded state. The cardiac phase may be determined as described in the previous example. In this example, the operation of registering the vessels may be performed based on a detected position of the balloon in the images in the live sequence. For instance, the balloon may be detected in the vessel, and the balloon registered to a position within a vessel in the volumetric image data 180. As described above, the balloon is visible in the live sequence of images by virtue of the presence of a contrast agent within the balloon. Alternatively, the operation of registering the vessels may be performed based on a detected position of a guidewire within the vessel in the images in the live sequence. In this case, the guidewire may be detected in the vessel, and the guidewire registered to a position within the vessel in the volumetric image data 180. A balloon is typically positioned in a vessel by translating the balloon along a guidewire, and the guidewire is visible in the live sequence of images by virtue of it radiopacity.

In general, the registration operation that is performed in this example may be rigid and/or elastic. In one example, the registration operation may be performed in accordance with the technique disclosed in the document WO2012/107857A1. The operation of generating the outputted information 160, 170 based on the registered vessels, may for instance include displaying the first and second graphical representations of the vessels. Additionally, or alternatively, it may include outputting a value of a metric 170 indicating a difference between the shape 140 of the balloon in the simulated expanded state and the shape 150 of the balloon 130 represented in the projection image data 120, as described above, using the spatially-aligned balloons.

In another example, the operation of outputting S120 information 160, 170 for comparing a state of expansion of the balloon 130 represented in the projection image data 120 with the simulated expanded state, comprises:
registering the vessel at the cardiac phase represented in the volumetric image data 180 to the vessel in the current images in the live sequence; and
generating the outputted information 160, 170 based on the registered vessels.

In this example, the registering operation has the effect of spatially aligning the balloon in the volumetric image data 180 to the balloon in the current images. In this example, the balloon may still move in the current images, however, the balloon in the simulated expanded state is also moved in accordance with this movement. Thus, it also reduces the impact of cardiac motion on the outputting, helping to provide a clearer comparison between the state of expansion of the balloon represented in the projection image data, and the simulated expanded state. As in the previous example, the registration operation may be rigid and/or elastic. The registration operation may be performed in accordance with the technique disclosed in the document WO2012/107857A1. The operation of generating the outputted information 160, 170 based on the registered vessels, may likewise include displaying the first and second graphical representations of the vessels. Additionally, or alternatively, it may include outputting a value of a metric 170 indicating a difference between the shape 140 of the balloon in the simulated expanded state and the shape 150 of the balloon 130 represented in the projection image data 120, as described above, using the spatially-aligned balloons.

In one example, the one or more processors 110 are configured to store the second graphical representation 150 of the balloon 130 generated for a current image in the live sequence. In this example, the operation of outputting a second graphical representation 150 of the balloon 130 comprises overlaying the stored second graphical representation of the balloon 130 with the second graphical representation 150 of the balloon 130 generated for the subsequent current images in the live sequence.

In this example, overlaying the stored second graphical representation of the balloon 130 with the second graphical representation 150 of the balloon 130 generated for the subsequent current images in the live sequence, enables changes in the actual state of expansion of the balloon to be compared over time. The stored second graphical representation of the balloon 130 may represent its state of expansion at any point in time. In one example, the stored second graphical representation of the balloon 130 represents its maximum state of expansion. As mentioned above, during an angioplasty procedure, a physician may expand and contract a balloon multiple times, each time inflating the balloon the same, or an increased, state of expansion. Here it may be useful to refer back to an earlier maximum state of expansion of the balloon.

In one example, the operation of simulating a shape of an expanded virtual balloon in the vessel comprises:
analyzing the projection image data 120 to identify a type of the balloon in the live sequence of images;
determining one or more balloon parameters for the type of the balloon in the live sequence of images; and
wherein the simulating a shape of an expanded virtual balloon in the vessel is performed using the one or more balloon parameters.

In this example, the balloon type, is determined from the projection image data, and corresponding balloon parameters, are used to simulate the shape of the expanded virtual balloon in the vessel. The balloon parameters may be extracted from a database, based on the identified balloon type. By automatically determining the balloon parameters, this example may save time because it may obviate the need to manually provide the balloon parameters. The type of the balloon may be determined from the projection image data using image processing techniques such as feature detection. Machine learning techniques, such as a neural network, for example, may also be used to identify the balloon type. As mentioned above, the balloon may include one or more radiopaque markers that serve to enhance its visibility. These markers may also be used to detect the position of the balloon, and to determine the balloon type.

It is noted that the system 100 may also include one or more of: a projection imaging system for providing the projection image data 120, such as for example the projection X-ray imaging system 230 illustrated in Fig. 1; a display, such as the display 240 illustrated in Fig. 1, for displaying the outputted information 160, 170, other outputs generated by the one or more processors 110, and so forth; a balloon, for example the balloon catheter 250 including the balloon 130 illustrated in Fig. 1; a patient bed 260; and a user input device (not illustrated in Fig. 1) configured to receive user input in relation to the operations performed by the one or more processors 110, such as a keyboard, a mouse, a touchscreen, and so forth.

In one example, a computer-implemented method of providing feedback during balloon angioplasty, is provided. The method comprises:
receiving S110 projection image data 120 representing a balloon 130 in a vessel;
outputting S120, based on a shape 140 of the balloon in a simulated expanded state in the vessel, and based on a shape 150 of the balloon 130 represented in the projection image data 120, information 160, 170 for comparing a state of expansion of the balloon 130 represented in the projection image data 120 with the simulated expanded state.

In one example, a computer program product is provided. The computer program product comprises instructions which when executed by one or more processors, cause the one or more processors to carry out a method of providing feedback during balloon angioplasty. The method comprises:
receiving S110 projection image data 120 representing a balloon 130 in a vessel
outputting S120, based on a shape 140 of the balloon in a simulated expanded state in the vessel, and based on a shape 150 of the balloon 130 represented in the projection image data 120, information 160, 170 for comparing a state of expansion of the balloon 130 represented in the projection image data 120 with the simulated expanded state.

The above examples are to be understood as illustrative of the present disclosure, and not restrictive. Further examples are also contemplated. For instance, the examples described in relation to a system, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing feedback during balloon angioplasty, the system comprising one or more processors (110) configured to:
receive (S110) projection image data (120) representing a balloon (130) in a vessel;
output (S120), based on a shape (140) of the balloon in a simulated expanded state in the vessel, and based on a shape (150) of the balloon (130) represented in the projection image data (120), information (160, 170) for comparing a state of expansion of the balloon (130) represented in the projection image data (120) with the simulated expanded state.

2. The system according to claim 1, wherein the one or more processors (110) are further configured to:
receive volumetric image data (180) representing the vessel; and
simulate, using the volumetric image data (180), a shape of an expanded virtual balloon (130^{V}) in the vessel to provide the shape (140) of the balloon in the simulated expanded state.

3. The system according to claim 1 or claim 2, wherein the shape (140) of the balloon in the simulated expanded state in the vessel represents the shape of the balloon in the simulated expanded state within a virtual stent in the vessel.

4. The system according to claim 2, wherein the simulating a shape of an expanded virtual balloon (130^{V}) in the vessel to provide the shape (140) of the balloon in the simulated expanded state, comprises:
simulating, using the volumetric image data (180), a rupture of a calcium deposit (190) within the vessel; and
determining the shape (140) of the balloon in the simulated expanded state based on the simulated rupture of the calcium deposit (190).

5. The system according to any one of claims 2-4, wherein the simulating a shape of an expanded virtual balloon (130^{V}) in the vessel comprises:
analyzing the volumetric image data (180) to identify i) a wall (210) of the vessel, and ii) one or more plaque components (190, 220) within the vessel;
assigning mechanical parameters to i) the wall (210) of the vessel, and to ii) each of the one or more plaque components (190, 220) within the vessel; and
determining the shape of the expanded virtual balloon (130^{V}) in the vessel based on i) the identified wall (210) of the vessel, and ii) the one or more plaque components (190, 220) within the vessel, using the assigned mechanical parameters.

6. The system according to any previous claim, wherein the outputting (S120) information (160, 170) for comparing a state of expansion of the balloon (130) represented in the projection image data (120) with the simulated expanded state, comprises:
outputting a first graphical representation (140) of the balloon wherein the balloon is in the simulated expanded state; and
outputting a second graphical representation (150) of the balloon (130) wherein the balloon is in the state of expansion represented in the projection image data (120).

7. The system according to claim 6, wherein the one or more processors (110) are further configured to output one or more further graphical representations of the balloon wherein the balloon is in one or more further simulated expanded states.

8. The system according to any one of claims 6-7 when dependent on claim 4, wherein the first graphical representation (140) of the balloon further represents the vessel, and wherein the first graphical representation (140) comprises a distinction between the wall (210) of the vessel and at least one of the one or more plaque components (190, 220).

9. The system according to any one of claims 6-8 when dependent on claim 3, wherein first graphical representation (140) comprises a virtual projection of the balloon (130^{VP}), and wherein the virtual projection of the balloon is generated by projecting the shape of the expanded virtual balloon (130^{V}) simulated using the volumetric image data (180) such that the balloon (130^{VP}) in the first graphical representation (140) is represented from a same viewing angle (α₁) as the balloon (130) in the second graphical representation (150).

10. The system according to any previous claim wherein the outputting (S120) information (160, 170) for comparing a state of expansion of the balloon (130) represented in the projection image data (120) with the simulated expanded state, comprises outputting a value of a metric (170) representing a difference between the shape (140) of the balloon in the simulated expanded state and the shape (150) of the balloon (130) represented in the projection image data (120).

11. The system according to any one of claims 3 - 10 wherein the one or more processors (110) are further configured to:
determine, based on the volumetric image data (180), a value of a recommended viewing angle (α₁) for acquiring the projection image data (120); and
output the value of the recommended viewing angle (α₁).

12. The system according to any previous claim wherein the one or more processors (110) are further configured to:
output an indication of a recommended type of a stent for use in stenting the vessel;
and/or
output an indication of a recommended value of a dimension of a stent for use in stenting the vessel.

13. The system according to any previous claim wherein the projection image data (120) comprises a live sequence of images representing the balloon (130) in the vessel, and wherein the outputting (S120) is performed for a current image in the live sequence and repeated for subsequent current images in the live sequence.

14. The system according to claim 13 when dependent on claim 3 wherein the volumetric image data (180) represents the vessel at a cardiac phase; and
wherein the outputting (S120) information (160, 170) for comparing a state of expansion of the balloon (130) represented in the projection image data (120) with the simulated expanded state, is performed selectively for current images from the live sequence representing the vessel at the cardiac phase represented in the volumetric image data (180);
or
wherein the outputting (S120) information (160, 170) for comparing a state of expansion of the balloon (130) represented in the projection image data (120) with the simulated expanded state, comprises:
registering the vessel at one or more different cardiac phases in the current images in the live sequence to the vessel at the cardiac phase represented in the volumetric image data (180); and
generating the outputted information (160, 170) based on the registered vessels;
or
wherein the outputting (S120) information (160, 170) for comparing a state of expansion of the balloon (130) represented in the projection image data (120) with the simulated expanded state, comprises:
registering the vessel at the cardiac phase represented in the volumetric image data (180) to the vessel in the current images in the live sequence; and
generating the outputted information (160, 170) based on the registered vessels.

15. The system according to claim 13 or claim 14 when dependent on claim 3, wherein the simulating a shape of an expanded virtual balloon in the vessel comprises:
analyzing the projection image data (120) to identify a type of the balloon in the live sequence of images;
determining one or more balloon parameters for the type of the balloon in the live sequence of images; and
wherein the simulating a shape of an expanded virtual balloon in the vessel is performed using the one or more balloon parameters.
